# EUROPÄISCHE PATENTSCHRIFT

(11) **EP 0 352 253 B1**
(45) Veröffentlichungstag und Bekanntmachung des Hinweises auf die Patenterteilung: **08.12.1993**
(21) Anmeldenummer: 89890193.9
(22) Anmeldetag: 18.07.1989
(51) Int. Cl.: A61F 2/68, F16H 3/54

(54) **Prothesenantrieb**
Servo motor for a prosthetic device
Dispositif de commande pour une prothèse

(30) Priorität: 18.07.1988 AT 1842/88
(43) Veröffentlichungstag der Anmeldung: 24.01.1990
(73) Patentinhaber: Otto Bock Orthopädische Industrie Besitz- und Verwaltungs-Kommanditgesellschaft, 37115 Duderstadt (DE)
(72) Erfinder: Horvath, Eduard, A-1070 Wien (AT)
(74) Vertreter: Casati, Wilhelm, Dipl.-Ing.

(56) Entgegenhaltungen:
- DE-A- 2 121 023

## Beschreibung

Die Erfindung betrifft einen Prothesenantrieb mit einem Planetengetriebe, das zwei Antriebe und einen Abtrieb aufweist. Bei Prothesenantrieben, insbesondere solchen zur Bewegung von Greifern, welche die natürliche Greifbewegung der Finger ausführen, besteht die Forderung, zwei Greifer, welche dem Daumen und dem mit dem Mittelfinger verbundenen Zeigefinger entsprechen, rasch aufeinander zu (mit geringem Moment) zu bewegen und, sobald der zu greifende Gegenstand von den beiden Greifern geklemmt wird, mit geringer Geschwindigkeit, jedoch großem Drehmoment weiter zu bewegen, um den Gegenstand sicher erfassen und manipulieren zu können. Weiters soll der Antrieb raumsparend ausgebildet sein, um die Prothese mit geringem Gewicht verwirklichen zu können und ihr ein ansprechendes Erscheinungsbild geben zu können. Erreicht wird dies mit einem Prothesenantrieb der eingangs erwähnten Art, wenn erfindungsgemäß drei innenverzahnte (äußere), koaxial zueinander angeordnete Sonnenräder mit zueinander unterschiedlichen Zähnezahlen vorgesehen sind, in die mindestens zwei Planetenräder eingreifen, von welchen jedes mit dem mittleren Sonnenrad, jedoch nur mit einem der dem mittleren Sonnenrad jeweils an einer Seite benachbarten Sonnenräder in Eingriff steht, wobei die Planetenräder alternativ von einem der beiden außenverzahnten (inneren) Sonnenräder antreibbar sind. Mit dieser Ausgestaltung gelingt es, die Greifer in optimaler Weise zu bewegen, d.h. rasch in die Greifstellung zu schwenken, und dann die notwendige hohe Griffkraft zu erzeugen. Die die Finger repräsentierenden Greifer sind an den Abtrieb angeschlossen. Der Unterschied in den Zähnezahlen der innenverzahnten Sonnenräder kann beliebig gewählt werden, unter Berücksichtigung des Verhältnisses der Module.

Eine weitere Ausführungsform des Prothesenantriebes sieht vor, daß mindestens eines der Planetenräder, gegebenenfalls jedoch beide Planetenräder, zweistufig (stufenförmig) ausgebildet ist bzw. sind. Damit läßt sich eine günstige Raumaufteilung der Getriebeelemente erreichen. Eine Veränderung der Übersetzung ist leicht durch entsprechende Stufenwahl zu verwirklichen und ein günstiger Wirkungsgrad zu erzielen. Es ist jedoch auch möglich, die Planetenräder einstufig, d.h. ohne daß sich die ein Planetenrad bildenden, in benachbarte, innenverzahnte Sonnenräder eingreifenden Räder hinsichtlich des Durchmessers voneinander absetzen, auszubilden.

Eine weitere Ausgestaltung der Erfindung zeichnet sich dadurch aus, daß das mittlere, innenverzahnte Sonnenrad einen Kopfkreisdurchmesser besitzt, der mit dem Kopfkreisdurchmesser von mindestens einem der dem mittleren Sonnenrad benachbarten, innenverzahnten Sonnenräder übereinstimmt. Diese Ausführungsform zeichnet sich durch besonders einfache Herstellbarkeit aus, die jedoch mit einer Verschlechterung des Wirkungsgrades verbunden ist.

Um ein gewünschtes Verhältnis zwischen den Drehzahlen der beiden Antriebe in einfacher Weise zu erreichen, kann vorgesehen werden, daß wenigstens einem der jeweils vom mittleren, innenverzahnten Sonnenrad und einem der ihm benachbarten, innenverzahnten Sonnenräder gebildeten Sonnenradpaare ein oder mehrere Vorstufen, die gegebenenfalls auch als Planetengetriebe ausgebildet sind, vorgeschaltet sind. Für die automatische Umschaltung von einem Antrieb auf den anderen kann eine, insbesondere elektronische, Schalteinrichtung für die beiden Antriebe vorgesehen sein, welche bei Erreichen eines Schwellenwertes des Stromes des einen Antriebes diesen stillsetzt, gleichzeitig jedoch den anderen Antrieb einschaltet.

Die Erfindung wird nachstehend anhand der Zeichnung näher erläutert. Es zeigen,
Fig. 1 einen Axialschnitt durch eine erste Ausführungsform eines Prothesenantriebes,
Fig. 2 einen Axialschnitt durch eine zweite Ausführungsform eines Prothesenantriebes,
Fig. 3 einen Schnitt entlang der Linie III-III in Fig. 1 und
Fig. 4 ein Blockschaltbild der elektrischen bzw. elektronischen Schaltung.

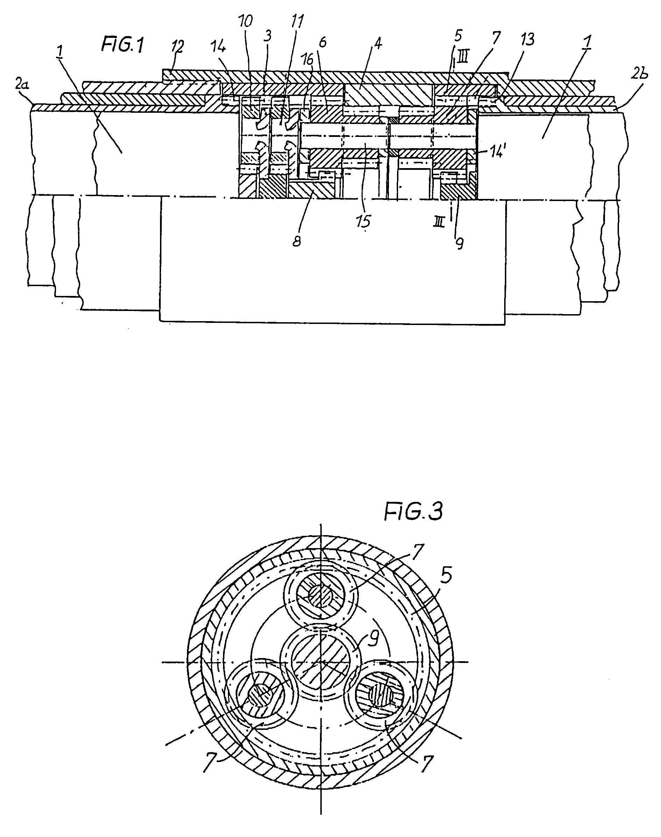

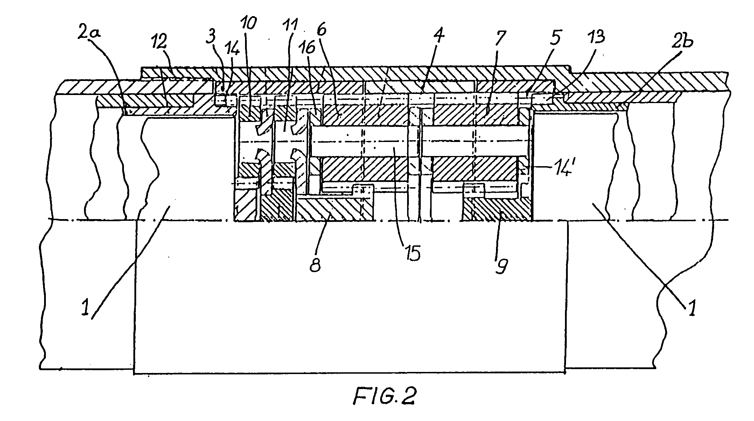

In der Zeichnung sind mit 1 zwei Antriebe bezeichnet, die koaxial zueinander angeordnet sind. Die Antriebe werden von Elektro-Kleinstmotoren gebildet. Das Planetengetriebe weist einen einzigen Abtrieb 2a und 2b auf, der hülsenförmig ausgebildet ist und die beiden Antriebe 1 umschließt. Am Abtrieb 2b kann dabei ein die Finger, nämlich den Zeige- und den Mittelfinger, repräsentierender Greifer und am Abtrieb 2a ein den Daumen repräsentierender Greifer angeschlossen sein. Das Planetengetriebe besitzt drei innenverzahnte (äußere) Sonnenräder 3, 4 und 5 mit zueinander unterschiedlichen Zähnezahlen. Die Sonnenräder 3, 4 und 5 sind dabei koaxial zueinander angeordnet. In die Sonnenräder 3, 4 und 5 greifen mindestens zwei Planetenräder 6 und 7 ein, von welchen jedes mit dem mittleren Sonnenrad 4, jedoch nur mit einem der dem mittleren Sonnenrad 4 jeweils an einer Seite benachbarten Sonnenräder 3, 5 in Eingriff steht. Die Planetenräder 6, 7 sind alternativ von einem der beiden außenverzahnten (inneren) Sonnenräder 8, 9 antreibbar. In dem in Fig. 1 dargestellten Ausführungsbeispiel sind beide Planetenräder 6, 7 zweistufig (stufenförmig) ausgebildet. Davon unterscheidet sich nun die in Fig. 2 dargestellte Ausführungsform des Prothesenantriebes, bei dem die beiden Planetenräder 6, 7 ohne Stufe ausgebildet sind. In dieser Ausführungsform besitzt das mittlere, innenverzahnte Sonnenrad 4 einen Kopfkreisdurchmesser, der mit dem Kopfkreisdurchmesser der beiden dem mittleren Sonnenrad 4 benachbart angeordneten, innenverzahnten Sonnenräder 3 und/oder 5 übereinstimmt. Bei der Ausführungsform gemäß Fig. 1 hingegen besitzt das mittlere, innenverzahnte Sonnenrad 4 einen Kopfkreisdurchmesser, der geringer ist als der Kopfkreisdurchmesser der dem mittleren, innenverzahnten Sonnenrad 4 benachbarten, ebenfalls innenverzahnten Sonnenräder 3 und 5.

Mit 10, 11 ist in den Ausführungsformen gemäß den Fig. 1 und 2 jeweils eine Vorstufe bezeichnet, die dem mittleren, innenverzahnten Sonnenrad 4 und dem diesem Sonnenrad benachbarten, innenverzahnten Sonnenrad 6 vorgeschaltet ist. Die Vorstufe ist in den beiden dargestellten Ausführungsformen als Planetengetriebe ausgebildet, jedoch kann auch ein anderes Getriebe als Vorstufe verwendet werden. Zum wahlweisen Schalten der beiden Antriebe 1 ist eine, insbesondere elektronische (nicht dargestellte), Schalteinrichtung vorgesehen, welche bei Erreichen eines Schwellenwertes des Stromes des einen Antriebes, z.B. des rechts gelegenen Antriebes, diesen stillsetzt, gleichzeitig jedoch den anderen Antrieb (der in der Zeichnung links dargestellt ist) einschaltet.

Das Planetengetriebe ist in einem hülsenförmigen Gehäuse 12 untergebracht. Der Abtrieb 2b ist über eine Steckkupplung 13 mit dem innenverzahnten Sonnenrad 5 gekuppelt, und der Abtrieb 2a ist in gleicher Weise über eine Steckkupplung 14 mit dem innenverzahnten Sonnenrad 3 gekuppelt.

Die außenverzahnten (inneren) Sonnenräder 8, 9 stehen entweder direkt mit dem Antrieb 1 (der von einem Elektromotor gebildet wird) in Verbindung, oder über die bereits erwähnte Vorstufe 10, 11, die vom Antrieb 1 betätigt wird.

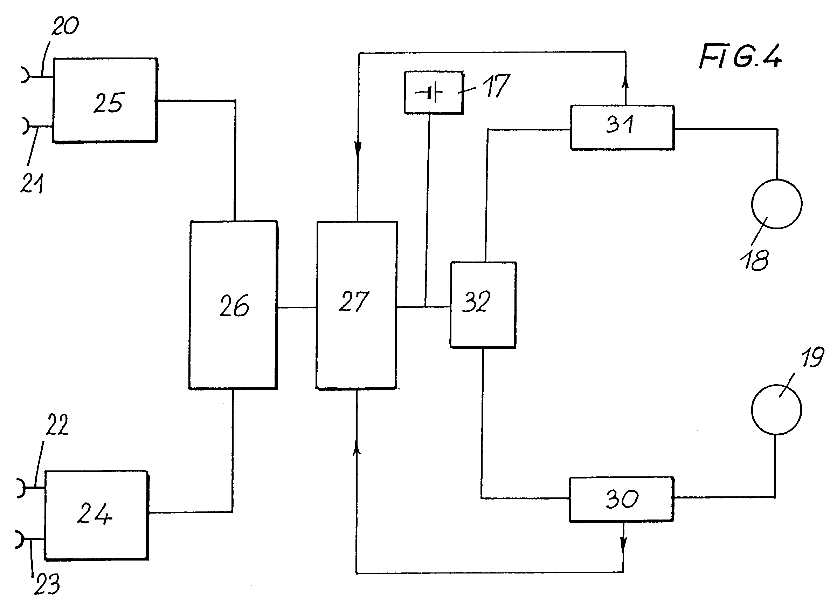

Wie Fig. 3 erkennen läßt, sind drei Planetenräder 7 innerhalb des innenverzahnten Sonnenrades 5 angeordnet. Analoges gilt hinsichtlich der Planetenräder 6, die ebenfalls zu dritt innerhalb des Sonnenrades 3 angeordnet sind. Die Planetenräder 7 sind in einem Käfig 14' drehbar gelagert. Zur Lagerung der Wellen 15 der Planetenräder 6 ist ein Käfig 16 vorgesehen. Der in der Zeichnung dargestellte Prothesenantrieb funktioniert wie folgt:
Wird der Motor 1, der sich in der Zeichnung rechts befindet, mit hoher Drehzahl in Betrieb genommen, so bewegen sich die mit dem Abtrieb 2a, 2b verbundenen Greifer schnell in Schließrichtung aufeinander zu. Der zweite Motor (in der Zeichnung links befindlich) steht dabei still. Sobald sich zwischen den beiden Greifern ein erhöhter Widerstand einstellt, der anzeigt, daß die beiden Greifer auf den zu ergreifenden Gegenstand aufgetroffen sind, erhöht sich der Strom im Antriebsmotor, wodurch auf elektronischem Weg eine Umschaltung auf den zweiten Antriebsmotor (in der Zeichnung links) erfolgt und der in der Zeichnung rechts befindliche Motor gleichzeitig stillgesetzt wird. Der Grenzstrom des in der Zeichnung links dargestellten Motors entspricht dabei der maximalen Haltekraft. Die Umsteuerung von der Schließbewegung der Greifer auf die Öffnungsbewegung kann entweder durch einen händisch betätigbaren Schalter oder aber über Muskelströme erfolgen.

Es ist sicherzustellen, daß im Betrieb immer nur ein Antrieb (Motor) in Tätigkeit ist, d.h. der andere Antrieb (Motor) feststeht, also auch kein Rücklauf dieses Motors erfolgt, z.B. durch Vorsehen einer Rücklaufsperre.

In Fig. 4 ist mit 17 eine Batterie zur wahlweisen Versorgung des Motors 18 oder des Motors 19 mit Spannung. Die Steuersignale werden von Hautelektroden 20, 21, 22, 23 geliefert. Die Signale werden in einer Schaltung 24 bzw. 25 aufbereitet, vor allem verstärkt und gleichgerichtet. Die Schaltungen 24, 25 liefern die aufbereiteten Signale an eine Komparatoren-Schaltung 26, deren Ausgang an den Eingang einer Auswahllogik 27, an welche ein den Strom in Leistungsstufen 30 oder 31 repräsentierendes Signal rückgemeldet wird. Der Ausgang der Auswahllogik ist an einen Umschalter 32 gelegt, der entweder die Leistungsstufe 30 oder 31 aktiviert, je nachdem, ob der Motor 19 oder der Motor 18 anzutreiben ist. Von den Motoren 18 und 19 steht jeweils einer still.

## Patentansprüche

1. Prothesenantrieb mit einem Planetengetriebe, das zwei Antriebe und einen Abtrieb aufweist, dadurch gekennzeichnet, daß drei innenverzahnte (äußere), koaxial zueinander angeordnete Sonnenräder (3, 4, 5) mit zueinander unterschiedlichen Zähnezahlen vorgesehen sind, in die mindestens zwei Planetenräder (6, 7) eingreifen, von welchen jedes mit dem mittleren Sonnenrad (4), jedoch nur mit einem der dem mittleren Sonnenrad jeweils an einer Seite benachbarten Sonnenräder (3, 5) in Eingriff steht, wobei die Planetenräder (6, 7) alternativ von einem von zwei außenverzahnten (inneren) Sonnenrädern (8, 9) antreibbar sind.

2. Prothesenantrieb nach Anspruch 1, dadurch gekennzeichnet, daß mindestens eines der Planetenräder (6 oder 7), gegebenenfalls jedoch beide Planetenräder, zweistufig (stufenförmig) ausgebildet ist bzw. sind.

3. Prothesenantrieb nach Anspruch 1, dadurch gekennzeichnet, daß das mittlere, innenverzahnte Sonnenrad (4) einen Kopfkreisdurchmesser besitzt, der mit dem Kopfkreisdurchmesser von mindestens einem der dem mittleren Sonnenrad (4) benachbarten, innenverzahnten Sonnenräder (3 und/oder 5) übereinstimmt.

4. Prothesenantrieb nach einem der Ansprüche 1 bis 3, dadurch gekennzeichnet, daß wenigstens einem der jeweils vom mittleren, innenverzahnten Sonnenrad und einem der ihm benachbarten, innenverzahnten Sonnenräder (3 oder 5) gebildeten Sonnenradpaare (3, 4 oder 4, 5) eine oder mehrere Vorstufen, die gegebenenfalls auch als Planetengetriebe (10, 11) ausgebildet ist bzw. sind, vorgeschaltet ist bzw. sind.

5. Prothesenantrieb nach einem der Ansprüche 1 bis 4, dadurch gekennzeichnet, daß eine, insbesondere elektronische, Schalteinrichtung für die beiden Antriebe vorgesehen ist, welche bei Erreichen eines Schwellenwertes des Stromes des einen Antriebes diesen stillsetzt, gleichzeitig jedoch den anderen Antrieb einschaltet.

## Claims

1. Prosthesis drive with a planet gear which has two drives and one output, characterized in that three internally toothed (outer) sun wheels (3, 4, 5) arranged coaxially with one another, with numbers of teeth which are different from one another, are provided, in which at least two planet wheels (6, 7) engage, each of which is in engagement with the central sun wheel (4) but only with one of the sun wheels (3, 5) adjacent on one side in each case to the central sun wheel, the planet wheels (6, 7) being drivable alternatively by one of two externally toothed (inner) sun wheels (8, 9).

2. Prosthesis drive according to Claim 1, characterized in that at least one of the planet wheels (6 or 7), but if appropriate both planet wheels, is or are of two-step (step-shaped) design.

3. Prosthesis drive according to Claim 1, characterized in that the central, internally toothed sun wheel (4) has a tip diameter which corresponds to the tip diameter of at least one of the internally toothed sun wheels (3 and/or 5) adjacent to the central sun wheel (4).

4. Prosthesis drive according to one of Claims 1 to 3, characterized in that at least one of the sun wheel pairs (3, 4 or 4, 5) formed in each case by the central, internally toothed sun wheel and one of the internally toothed sun wheels (3 or 5) adjacent to it is preceded by one or more preliminary steps which is or are if appropriate also designed as planet gears (10, 11).

5. Prosthesis drive according to one of Claims 1 to 4, characterized in that an in particular electronic switching device is provided for the two drives, which, when a threshold value of the current of one drive is reached, switches it off but simultaneously switches the other drive on.

## Revendications

1. Entraînement de prothèse, avec une transmission planétaire, présentant deux entraînements et une partie entraînée, caractérisé en ce que sont prévues trois roues solaires (extérieures) (3, 4, 5), à denture intérieure, disposées coaxialement les uns par rapport aux autres, avec des nombre de dents différents entre les roues, avec lesquelles engrènent au moins deux roues planétaires (6, 7), dont chacune engrène avec la roue solaire centrale (4), mais cependant seulement avec l'une des roues solaires (3,5) voisines située d'un côté, les roues planétaires (6,7) étant susceptibles d'être entraînées alternativement par l'une des deux roues solaires (intérieures) (8,9) à denture extérieure.

2. Entraînement de prothèse selon la revendication 1, caractérisé en ce qu'au moins l'une des roues planétaires (6 ou 7), le cas échéant cependant les deux roues planétaires est/sont à deux étages (en forme étagée).

3. Entraînement de prothèse selon la revendication 1, caractérisé en ce que la roue solaire centrale (4) à denture intérieure comporte un diamètre de cercle de tête coïncidant avec le diamètre de cercle de tête d'au moins l'une des roues solaires (3 et/ou 5) à denture intérieure, voisines de la roue solaire centrale (4).

4. Entraînement de prothèse selon l'une des revendications 1 à 3, caractérisé en ce qu'au moins l'un des couples de roue solaires (3,4 ou 4,5) formé chaque fois par la roue solaire centrale à denture intérieure et l'une des roues solaires (3 ou 5) à denture intérieure, leur étant voisine, est/sont mise(s) en circuit en amont d'un ou plusieurs étages antérieurs, réalisés, le cas échéant, également sous forme de transmission planétaire (10,11).

5. Entraînement de prothèse selon l'une des revendications 1 à 4, caractérisé en ce qu'un dispositif de commutation, en particulier électronique, est prévu pour les deux entraînements, mettant à l'arrêt un entraînement lorsqu'est atteinte une valeur de seuil du courant pour cet entraînement, en mettant cependant simultanément l'autre entraînement en service.
